# EUROPEAN PATENT SPECIFICATION

(11) **EP 2 674 153 B1**
(45) Date of publication and mention of the grant of the patent: **10.08.2016**
(21) Application number: 12004493.8
(22) Date of filing: 14.06.2012
(51) Int. Cl.: A61K 9/20, A61K 31/54, A61K 33/14, A61K 45/06, A61K 31/549, A61K 9/24

(54) **Oral dosage form for the immediate release of bendroflumethiazide and controlled release of a second active agent**
Orale Darreichungsform zur schnellen Freisetzung von Bendroflumethiazid und zur kontrollierten Freisetzung eines zweiten Wirkstoffes
Compositions pharmaceutiques à voie orale pour la libération immédiate de bendroflumethiazide et pour la libération contrôlée d'un deuxième agent actif

(43) Date of publication of application: 18.12.2013
(73) Proprietor: ratiopharm GmbH, 89079 Ulm (DE)
(72) Inventor: Genth, Maria, Dr., 89077 Ulm (DE); Scheiwe, Max-Werner, Dr., 79689 Maulburg (DE)
(74) Representative: Ter Meer Steinmeister & Partner

(56) References cited:
- EP-A2- 0 106 443
- WO-A1-2009/084040
- US-A1- 2003 152 622

## Description

The present invention relates to an oral dosage form for the immediate release of bendroflumethiazide and controlled release of a second active agent, and a method for its preparation.

Bendroflumethiazide is the international nonproprietary name (INN) for 3-benzyl-1,1-dioxo-6-(trifluoromethyl)-3,4-dihydro-2*H*-1,2,4-benzothiadiazine-7-sulfonamide (IUPAC), which is represented by the formula below:

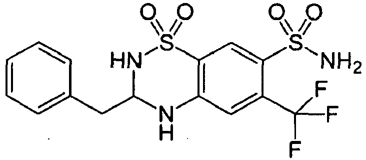

Bendroflumethiazide is a thiazide diuretic used to treat hypertension and oedema. It can advantageously be administered with further active agents. Bendroflumethiazide is supposed to remove excess water from a patient's body by causing an increase in the removal of electrolytes (salts) from the patient's blood, thus drawing water out of the blood and into the kidneys. This causes a drop in the volume of blood being transported around the body, which in turn lowers blood pressure. However, the removal of electrolytes may lead to undesirable side effects. Therefore, replenishing electrolytes is advantageous, if not necessary. This replenishment should ideally take place some time after ingestion of bendroflumethiazide in order to avoid diminishing its efficacy. In order to facilitate administration to the patient and avoid a complicated dosage regimen involving the need for taking different medications at predetermined time intervals, an oral dosage form should therefore ideally provide for immediate release of bendroflumethiazide and controlled release of electrolyte.

EP 0 106 443 discloses a pharmaceutical oral controlled release multiple-units formulation in which individual units comprise coated units containing an active substance which is subject to controlled release as a result of coating of the units with a water-insoluble, but water diffusable controlled release coating, the units additionally comprising particles of an active substance adhered to the surface of the controlled release coating in a substantially uniform layer. Controlled release coated KCl units may be combined with an instant release diuretic, such as bendroflumethiazide.

WO 2009/084040 discloses once a day formulations of angiotensin receptor blockers (ARBs). The formulations comprise solubilized ARB and are in the form of gastroretentive dosage forms. Bendroflumethiazide is mentioned amongst therapeutic agents which may be combined with an ARB.

US 2003/0152622 discloses a controlled release oral dosage form for the continuous, controlled administration of a diuretic agent to the stomach, duodenum and upper sections of the small intestine of a patient, the dosage form comprising a matrix having the active agent dispersed therein.

Currently, there are tablets on the market, which contain bendroflumethiazide together with potassium chloride. They are marketed under the tradename Centyl K®. These tablets have a tablet core comprising potassium chloride and ethyl cellulose, amongst others, and provide for controlled release of the potassium chloride. Bendroflumethiazide is comprised in a coating around the tablet core, which coating, amongst others, further comprises polysorbate 20. Polysorbate 20 is presumed to act as a solubility enhancer for bendroflumethiazide, which is practically insoluble (according to Ph. Eur., indicating a solubility in the range of 10 to 100 mg/l). Polysorbate 20 is a short term for polyoxyethylene (20) sorbitan monolaurate.

However, there is an issue with the stability of dosage forms comprising bendroflumethiazide and a further pharmaceutically active agent, such as KCl, in particular upon storage. It has been found that bendroflumethiazide tends to degrade when combined with polysorbate or other polyethoxylated polymers. It has further been found that potassium chloride accelerates degradation of bendroflumethiazide.

Therefore, a need exists for an oral dosage form comprising bendroflumethiazide in combination with a further active agent, which active agent may cause degradation of bendroflumethiazide, which has improved stability, in particular upon storage.

Also, a need exists for an oral dosage form comprising bendroflumethiazide, advantageously in combination with a further active agent, which provides for good dissolution of bendroflumethiazide in the gastrointestinal tract.

The present invention provides an oral dosage form for the immediate release of bendroflumethiazide and controlled release of a second active agent, the dosage form comprising a first portion comprising bendroflumethiazide, wherein the first portion is adapted for immediate release of bendroflumethiazide, and a second portion comprising the second active agent, the second portion being separate from the first portion and adapted for controlled release of the second active agent, wherein the oral dosage form is free from polyethylene oxide and polymers comprising polyethylene oxide moieties and preferably free from polyalkylene oxides and polymers comprising polyalkylene oxide moieties.

Bendroflumethiazide may be present in the form depicted above or in the form of any pharmaceutically acceptable salt or solvate. Any reference to an amount of bendroflumethiazide herein shall be understood as referring to the molecule depicted above.

Polyethylene oxides are polymers designated by the general formula H-(O-CH₂-CH₂)ₙ-OH. Preferably, in the polyethylene oxides H-(O-CH₂-CH₂)ₙ-OH or polyethylene oxide moieties -(O-CH₂-CH₂)ₙ- referred to in the context of the present invention, n is at least 10, further preferably n is at least 5 and in further embodiments, n may be 2 or more. The same considerations concerning n apply to polyalkylene oxides H-(O-R)ₙ-OH and polymers comprising polyalkylene oxide moieties -(O-R)ₙ with R being alkyl, preferably C₃ to C₁₀₀ alkyl and more preferably C₃ to C₁₀ alkyl.

The term "free from polyethylene oxide and polymers comprising polyethylene oxide moieties or more generally polyalkylene oxides and polymers comprising polyalkylene oxide moieties", as used herein, means that polyethylene oxide, respectively polyalkylene oxides, and polymers comprising polyethylene oxide moieties, respectively polymers comprising polyalkylene oxide moieties, may be present only in trace amounts that do not have any detrimental effect upon the stability of the bendroflumethiazide. If present, these compounds should therefore be contained in amounts of less than 7.5 mg per oral dosage form, more preferably less than 5, 4, 3, 2, 1 or 0.5 mg per oral dosage form and most preferably not be contained at all. Exemplary polymers comprising polyethylene oxide moieties are polysorbates, which are marketed under the trade name Tween®. Such polymers are also often part of commercially available and commonly used film formers, such as Opadry®. One of the commonly used typical Opadry® II White formulations, for instance, contains polyvinyl alcohol, titanium dioxide, talc, polyethylene glycol 3350, and lecithin. However, there are also Opadry® II White formulations available, which do not contain this type of polymer.

The oral dosage form according to the present invention is adapted for the immediate release of bendroflumethiazide. This means that the release profile of the dosage forms of the invention according to USP method (basket, 500 ml, 0.01 n HCl, 125 rpm, 37 °C) usually indicates a content release of at least 80 % bendroflumethiazide after 60 minutes, for instance at least 80 % bendroflumethiazide after 30 minutes or at least 80 % bendroflumethiazide after 20 minutes.

The oral dosage form according to the present invention is adapted for the controlled release of a second active agent. This means that the release profile of the dosage forms of the invention according to USP method (basket, 900 ml, phosphate buffer pH 6.8, 75 rpm, 37 °C) usually indicates a content release of at most 30 % or 40% of the second active agent after one hour, for instance at most 25 % after one hour, preferably at most 60 % at most 50 % or at most 45 % after 2 hours, and for instance 30 to 55 % after 2 hours or 35 to 50 % after 2 hours.

The oral dosage form according to the present invention is suitable and adapted for oral administration by ingestion (swallowing unchewed).

The second active agent is a pharmaceutically active agent other than bendroflumethiazide. The oral dosage form according to the present invention is particularly advantageous in those embodiments wherein the second active ingredient is potassium chloride KCl. Alternative second active agents comprise one or more of an ACE inhibitor, such as captopril, enalapril, lisinopril, fosinopril, zofenopril, cilazapril, ramipril, benazepril, moexipril, quinapril, perindopril, and trandolapril, an alpha-adrenergic agonist, such as methyldopa, an angiotensin II receptor antagonist, such as almesartan, valsartan, irbesartan, candesartan, telmisartan, eprosartan, and losartan, a beta blocker, such as propranolol, carvedilol, metoprolol, bisoprolol, nebivolol, bevantolol, timolol, labetalol, pindolol, atenolol, celiprolol, talindolol, and betaxolol, an antihypertensive drug, such as guanethidine, and reserpine, a renin inhibitor, such as aliskiren, and a calcium channel blocker, such as nifedipine, nimodipine, nitrendipine, amlodipine, felodipine, nisoldipine, lercanidipine, verapamil, and dilthiazem.

In particularly preferred embodiments of the present invention, the first portion of the oral dosage form comprises a surface-active agent having an HLB value of 12 or more. The HLB value may be 13 or more, 14 or more, or from 15 to 16, for instance. The HLB value characterizes the hydrophilic-lipophilic balance of molecules. The term HLB value as used herein is in accordance with the general understanding of the person skilled in the art (as defined in Fiedler, Lexikon der Hilfsstoffe, 5th edition, 2002, for instance, including the analytical methods cited therein). The surface-active agent serves as a solubility enhancer for bendroflumethiazide and thus improves the bioavailability of the active ingredient.

Preferably, the surface-active agent is a sucrose ester (with an HLB value of 12 or more). Sucrose esters are mono-, di-, tri- and polyesters of sucrose (C₁₂H₂₂O₁₁), in particular with fatty acids. Sucrose esters may be prepared, for instance, by transesterification of methyl and ethyl esters of fatty acids with sucrose or by extraction from sucroglycerides. The sucrose ester may be a single sucrose ester or mixture of sucrose esters. The term "sucrose ester" as used herein encompasses both individual esters as well as mixtures thereof. In case of a mixture, the HLB value refers to the HLB value of the mixture.The fatty acid is preferably selected from straight-chain or branched fatty acids having 5 carbon atoms or more, more preferably 8 carbon atoms or more, more preferably 10 carbon atoms or more, and preferably up to 30 carbon atoms, more preferably up to 24 carbon atoms, and particularly preferred 16 or 18 carbon atoms. Preferably, the sucrose ester used herein is or predominantly comprises a monoester, i.e. an ester that combines 1 mol of fatty acid with 1 mol of sucrose. In preferred embodiments, the one or more monoesters make up at least 50, preferably at least 60 and more preferably 70 percent by weight of the ester composition. Preferred sucrose esters are sucrose palmitate, sucrose laurate and sucrose stearate, with sucrose palmitate being particularly preferred. The term sucrose palmitate implies that palmitate makes up at least 50% of the fatty acid comprised in the sucrose ester. The same considerations apply *mutatis mutandis* to the terminology of other esters. For a given sucrose ester, such as sucrose stearate, which is a mixture of sucrose esters, the HLB value can be varied through appropriate choice of the relative amounts of mono-, di-, tri- and polyesters of a given fatty acid and/or the relative amount of a certain fatty acid. This is exemplified by the following table of commercially available sucrose esters:

| | trade name | HLB | percentage of main fatty acid | ester composition % | |
|---|---|---|---|---|---|
| | | | | mono | di, tri, poly |
| sucrose stearate | Surfhope SE 1811 | 11 | 70 | 55 | 45 |
| | Surfhope SE D-1815 | 15 | 70 | 70 | 30 |
| | Surfhope SE D-1816 | 16 | 70 | 75 | 25 |
| sucrose palmitate | Surfhope SE D-1615 | 15 | 80 | 70 | 30 |
| | Surfhope SE D-1616 | 16 | 80 | 80 | 20 |

The surface-active agent with an HLB value of 12 or more may be comprised in an amount of from 1, 2, 3, 4, 5, 6, 7, 8, 9 or 10 parts by weight relative to 1, 2, 3, 4, 5, parts by weight of bendroflumethiazide, for instance, such as ratios of 1:1, 1:2 , 4:1 or 2:1 parts of surface-active agent relative to bendroflumethiazide.

It has been found that the oral dosage forms according to the present invention show good dissolution of bendroflumethiazide and improved stability.

Most preferably, the oral dosage form is a tablet. The first portion is preferably a film coating. In those embodiments, the remainder of the tablet containing the second active agent, preferably KCl (the second portion), is coated with a film containing bendroflumethiazide (the first portion). The terms film, film coating and coating are used synonymously herein.

The second portion of the oral dosage form comprises a controlled release agent for controlling the release of the second active agent.

The second active agent may be comprised in the oral dosage form, more specifically in the second portion thereof, in the form of particles, which particles are coated with a controlled release agent such that the coated particles release the second active agent in a controlled manner.

Suitable embodiments of second portions according to the present invention, in particular second portions of tablets according to the present invention, comprise or consist of the following:
- "CR matrix":
   The second portion is a tablet core, wherein the second active agent is comprised, preferably homogeneously dispersed, in a controlled release matrix comprising a controlled release agent. This may be referred to as the matrix embodiment or controlled release matrix embodiment in the following. In those embodiments, the film coating comprising the bendroflumethiazide may be disposed directly on the tablet core, preferably continuously surrounding the tablet core, i.e. without intermediate film coating or intermediate layer. However, in alternative embodiments, there may be an additional film coating disposed between the tablet core and the bendroflumethiazide containing film coating, which, however, preferably does not influence the release of the second active agent to any considerable extent.
- "CR matrix + CR film":
   The second portion comprises a tablet core, wherein the second active agent is comprised in a controlled release matrix comprising a controlled release agent. The tablet core is further coated with a film comprising a controlled release agent. Preferably, the (CR) film does not comprise the second active agent. Both the film and the matrix control the release of the second active agent.

### - "IR core/CR film"

The second portion comprises a tablet core and a film coating disposed on the tablet core, preferably completely surrounding the tablet core. The tablet core comprises the second active agent, and the film coating comprises a controlled release agent, but preferably no second active agent. Preferably, the tablet core itself is an immediate release tablet core, *i.e.* does not substantially delay release of the second active agent. The film coating is adapted to control release of the second active agent.

### - "Coated particles"

The second portion comprises a tablet core comprising particles of the second active agent. The particles of the second active agent are coated with a (first) controlled release agent. In a first embodiment, the tablet core does not comprise any further controlled release agent(s) and is directly coated with the bendroflumethiazide comprising film. In a second embodiment, the tablet core either comprises a further (second) controlled release agent, which may be the same or different from the controlled release agent coated around the particles (CR particles in CR matrix), or is coated with a film comprising a further (second) controlled release agent, which may be the same or different from the controlled release agent coated around the particles (CR particles in IR tablet core with CR coating), or a combination of both. Preferably, the first controlled release agent is different from the second controlled release agent.

In preferred embodiments, in particular in those wherein the second portion is a tablet core coated with a film comprising a controlled release agent, the second active ingredient may be provided in a mixture with at least one pharmaceutically acceptable excipient. Expressed differently, the tablet core comprises the second active agent and at least one pharmaceutically acceptable excipient.

Embodiments wherein particles of the second active agent or the tablet core are coated with a controlled release agent are particularly advantageous in that they allow dissolution of the second active agent to take place with a delay to the dissolution of bendroflumethiazide, thus avoiding any interaction between the active agents. This is particularly beneficial in cases like that of KCl, where the second active agent has a negative impact on the stability or more generally bioavailability of bendroflumethiazide.

A combination of controlled release coating and controlled release matrix allows fine tuning of the release characteristics. For instance, a delay in release combined with extended release may readily be achieved by a suitable combination of controlled release agents in coating and matrix.

Controlled release agents are well known in the art. Suitable controlled release agents may generally be selected, for instance, from polymers based on at least one of acrylate and methacrylate (commercially available as Eudragit^{®}), cellulose ethers and esters, such as ethylcellulose, hydoxypropylmethylcellulose, waxes and lipids, cellulose acetate phthalate (CAP), hydroxypropyl methylcellulose phthalate (HPMCP), hydroxypropyl methylcellulose acetate succinate (HPMC-AS) and polyvinyl acetate phthalate (PVAP).

Depending on whether the controlled release agent is present in a matrix or in a film coating, different controlled release agents may be preferable.

Polymers based on at least one of acrylate, in particular ethyl acrylate, and methacrylate (commercially available as Eudragit^{®}) are particularly preferred for coating particles of the second active agent. The polymers or copolymers may be neutral, anionic, cationic or zwitterionic, i. e. contain quarternary ammonium groups, and include examples and grades generally mentioned below in conncection with film coatings. A particularly preferred embodiment is a neutral copolymer of acrylate and methacrylate, and particularly preferred is poly(ethyl acrylate-co-methyl methacrylate), for instance Eudragit^{®} NM30D (poly(ethyl acrylate-co-methyl methacrylate) 2:1, approx. 600,000 g/mol).

If the second portion of the oral dosage form, such as a tablet, comprises the controlled release agent in the form of a coating, the coating may be chosen from so-called gastric juice resistant film-coatings and retard coatings, for instance. The following examples are preferred in particular in those embodiments wherein a tablet core is coated with a controlled release film.

In gastric juice resistant coatings, the solubility of the coating depends on the pH of the surrounding. Examples of gastric juice 'resistant' coatings are those that are based on polymers such as cellulose acetate phthalate (CAP), hydroxypropyl methylcellulose phthalate (HPMCP), hydroxypropyl methylcellulose acetate succinate (HPMC-AS), and polyvinyl acetate phthalate (PVAP), to name but a few controlled release agents enabling gastric juice resistance.

A further example of favourable controlled release agents in particular for controlled release film coatings are polymers or copolymers based on one or more of acrylate and methacrylate. The polymers or copolymers may be neutral, anionic, cationic or zwitterionic, *i.e.* contain quarternary ammonium groups. Cationic copolymers based on dimethylaminoethyl methacrylate and neutral methacrylic esters, such as polybutyl methacrylate, (2-dimethylaminoethyl)methacrylate, methacrylate 1:2:1 (Eudragit^{®} E grades) are exemplary embodiments that may be used. Further exemplary embodiments include ammonio methacrylate copolymer, type A (USP) (Eudragit^{®} RL grades) and ammonio methacrylate copolymer, type B (USP) (Eudragit^{®} RS grades). Particularly preferred is poly(ethyl acrylate-co-methyl methacrylate), for instance Eudragit^{®} NM30D (poly(ethyl acrylate-co-methyl methacrylate) 2:1, approx. 600,000 g/mol). Exemplary preferred controlled release agents for the controlled release coating include anionic acrylate and/or methacrylate based polymers and copolymers (commercially available e.g. as Eudragit^{®} L and S types). These polymers include or are copolymers based on ethyl or methyl esters of acrylic or methacrylic acid and methacrylic acid.

Retard coatings are usually non-soluble (preferably having a water solubility at 25 °C of less than 10 mg/ml). Examples of retard coatings comprise ethyl cellulose (EC, commercially available e.g. as Surelease^{®}) and acrylate and/or methacrylate based polymers and copolymers, such as cationic or neutral polymers and copolymers, as mentioned above.

Preferred controlled release agents for use in embodiments comprising a controlled release matrix are cellulose ethers and esters, more preferably cellulose ethers. Hydroxypropylmethylcellulose (HPMC) is particularly preferred. For this purpose, hydroxypropylmethylcellulose (HPMC) preferably has a methoxy group content of 15 to 30 % by weight, more preferably 19 to 24% by weight and/or a hydroxypropyl content of 5 to 20, more preferably 7 to 12 % by weight. Such HPMC grades are available under the tradename METHOCEL from Dow Chemical, for instance. As mentioned before, the matrix may further be coated with a controlled release agent, which may be the same or preferably different from the controlled release agent in the matrix. Suitable controlled release agents have been mentioned above.

In those embodiments wherein the second active agent is KCl, KCl crystals are suitably used as KCl particles. Preferably, KCl particles have a D50 value in the range of 100 to 400 µm.

The D50 volume distribution is defined in the context of this invention as the particle diameter, at which 50 percent by volume of the particles have a smaller diameter than the diameter, which corresponds to the D50 value. Likewise, 50 percent by volume of the particles have a larger diameter than the D50 value. Within this application, the particle size distribution may be determined by the light scattering method, for instance using a Mastersizer 2000 apparatus made by Malvern Instruments, preferably using dry measurement, such as by using dry powder dispersion unit Scirocco 2000(A) (exemplary settings: dispersant: air, dispersant pressure: 1.5 bar; Mie scattering; assumed values for absorption of 0.1 and refractive index (particles) of 1.52).

A first preferred embodiment of the present invention is a tablet with a tablet core comprising particles of the second active agent, preferably KCl, which are coated with a controlled release agent, preferably a polymer based on at least one of acrylate and methacrylate, which tablet core is coated with a film comprising bendroflumethiazide. Said film is adapted for immediate release of the bendroflumethiazide and preferably comprises a surface-active agent with an HLB value of more than 12 as a solubility enhancer, preferably a sucrose ester. There is no additional coating between the tablet core and the film comprising bendroflumethiazide. Those embodiments are advantageous in that they completely separate the second active agent and the bendroflumethiazide. They further allow delaying dissolution of the second active agent and thus enable sequential administration of bendroflumethiazide and the second active agent in a single tablet, whilst avoiding any interaction between the active agents, both in the tablet and in the patient's gastrointestinal tract. In particular, an initial burst of the second active agent can be avoided.

In a second preferred embodiment, the tablet comprises a tablet core wherein the second active agent, preferably KCl, together with at least one pharmaceutically acceptable excipient form the tablet core, for instance prepared by compressing KCl and the at least one pharmaceutically acceptable excipient. The tablet core is coated with a controlled release film for controlling the release of the second active agent. The film itself, however, is ideally free of the second active agent or more generally, any active agent. Thus, an initial burst of the second active ingredient is avoided. The tablet further comprises bendroflumethiazide in a film coated onto the controlled release film. As in the previous embodiment, said film is adapted for immediate release of the bendroflumethiazide and preferably comprises a surface-active agent with an HLB value of 12 or more as a solubility enhancer, preferably a sucrose ester.

A third preferred embodiment varies from the second preferred embodiment only in that the tablet core comprises a controlled release agent, making it a controlled release matrix. The tablet core is further coated with a controlled release film, as described for the second preferred embodiment. As mentioned before, those embodiments are particularly advantageous in that they allow fine adjustment of release characteristics and complete physical separation of the second active agent from the bendroflumethiazide.

The oral dosage forms, in particular tablets, of the present invention, may contain pharmaceutically acceptable excipients as common in the art. For instance, they may contain fillers/binders, lubricants, glidants, film forming agents, plasticizers, pH adjusting agents and/or colourants.

Fillers/binders, lubricants and/or glidants are typically used in the tablet core whereas film forming agents, plasticizers, pH adjusting agents and/or colourants may advantageously be used in the (film) coatings, in particular the bendroflumethiazide containing coating.

Fillers are understood herein to mean substances which are usually described as pharmaceutical fillers or filling agents and can also be referred to as constituents, extenders or basic materials. These fillers are typically substances used to form the body, or mass, of the oral dosage form in the case of dosage forms with small amounts of active agent, so as to obtain a sufficient amount of dosage form mass for a suitable dosage form size. Binders are understood herein as substances used to increase the strength of a tablet core or tablet, respectively, and/or granules comprised in the tablet core. Since many fillers also act as binders, these substances are treated together herein.

Fillers/binders for the purposes of the invention are, for example: lactose, lactose derivatives, starch, starch derivatives, treated starch such as pregelatinized starch, chitin; cellulose and derivatives thereof, e.g. microcrystalline cellulose, calcium phosphates, sucrose, calcium carbonate, magnesium carbonate, magnesium oxide, maltodextrin, calcium sulphate, dextrates, dextrin, dextrose, hydrogenated vegetable oil, kaolin, sodium chloride, potassium chloride and mixtures thereof can be used. Fillers/binders are generally used in an amount of 0.5 to 50 % by weight, for instance 1 to 25% by weight. based on the total weight of the tablet core.

Lubricants are generally used in order to reduce sliding friction during tablet compression between the punch moving in the die and the die wall, on the one hand, and between the edge of the tablet and the die wall, on the other hand. Suitable lubricants include talc, in particular micronized talc, kaoline, glycerol monostearate, monoglycerides, diglycerides, triglycerides, calcium stearate, magnesium stearate, stearic acid, adipic acid, sodium stearyl fumarate, silica, and the like. Talc is preferred. Lubricants are generally used in an amount of 0.1 to 3 % by weight, based on the total weight of the dosage form.

The task of glidants is to reduce both the interparticular friction (cohesion) between individual particles and their adherence to the wall surfaces of manufacturing equipment (adhesion). A preferred example of an additive to improve powder flowability is silica (e.g. Syloid^{®}). Glidants are generally used in an amount of 0.1 to 3 % by weight, based on the total weight of the dosage form.

Film forming agents are supposed to provide the base for a film coating without substantially affecting the release rate. Preferred examples of film forming agents, which enable immediate release of bendroflumethiazide, include hydroxypropyl methylcellulose (HPMC), methylcellulose (MC), hydroxypropyl cellulose (HPC), hydroxyethyl cellulose (HEC), carboxymethylcellulose sodium (CMC-Na), polyvinyl alcohol (PVA), poly(meth)acrylate, in particular cationic polymethacrylates with quarternary ammonium moieties (Eudragit^{®} E), preferably those having a molecular wieght between 135,000 and 150,000 g/mol, derivatives thereof, and mixtures thereof. Hydroxypropyl methylcellulose (HPMC) and hydroxyethylcellulose (HEC) can have an average molecular weight of 10,000 to 150,000 g/mol, for instance. Methylcellulose (MC) can have an average molecular weight of 20,000 to 150,000 g/mol, for instance. Hydroxypropyl cellulose (HPC) can have an average molecular weight of 60,000 to 1,200,000 g/mol, for instance. Carboxymethylcellulose sodium (CMC-Na) can have an average molecular weight of 80,000 to 600,000 g/mol, for instance and polyvinyl alcohol (PVA) can have an average molecular weight of 15,000 to 200,000 g/mol, for instance. Film forming agents may be used in an amount of 0.5 to 5 % by weight, such as 1 to 4 % by weight, for instance 2 % by weight, based on the total weight of the dosage form.

Suitable plasticizers include 1,2-propylene glycol, acetyl triethyl citrate, dibutyl phthalate, dibutyl sebacate, triacetine, triethyl citrate, polydextrose, maltodextrin and the like. Plasticizers are generally used in an amount of 10 to 40 % by weight, based on the weight of the film forming agent.

Suitable pH adjusting agents include citric acid, lactic acid, tartaric acid, fumaric acids, salts of these acids and mixtures thereof. PH adjusting agents may be used in an amount of 30 to 300 % by weight, based on the weight of the active ingredient.

The thickness of a film for the immediate release of bendroflumethiazide, such as coated around a tablet core, may range from 1 to 80 µm, for instance, preferably from 4 to 60 µm, for instance from 5 to 50 µm.

Preferably, the tablets, in particular the tablet cores, of the present invention have a hardness of from 100 to 300 Newton (N), more preferably from 170 to 270 N, wherein the hardness is measured according to Ph.Eur. 7.0, Chapter 2.9.8.

Further, the tablets of the present invention preferably have a friability of less than 3 %, more preferably of less than 2 %, still more preferably less than 1 %, in particular, 0.9 to 0.1 %, wherein the friability is measured according to Ph.Eur. 7.0, Chapter 2.9.7.

Further, the tablets of the present invention usually have a *Content Uniformity* from 95 to 105 %, preferably from 98 to 102 %, in particular from 99 to 101 % (That means, all tablets have a drug content from 95 to 105 %, preferably from 98 to 102 %, in particular from 99 to 101 %, wherein the average drug content of all tablets is set as 100 %). The *Content Uniformity* is determined according to Ph. Eur.7.0, Chapter 2.9.6.

In this context, it is generally noted that, due to the nature of pharmaceutical excipients, it cannot be excluded that a certain compound meets the functional requirements of more than one of the above mentioned excipient classes. However, in order to enable an unambiguous distinction and terminology in the present application, the same pharmaceutical compound can only be subsumed as one of the functional excipient classes presented above, with the exception of fillers/binders, which are treated as one common class herein. For example, if a substance is described as a glidant, it cannot additionally classify as a lubricant.

The oral dosage forms according to the present invention are for the treatment of hypertension and/or oedema.

Bendroflumethiazide may be comprised in amounts of from 1 to 50 mg per oral dosage form, for instance, such as in an amount of 1.25 mg, 2.5 mg or 5 mg (based on the content of bendroflumethiazide as illustrated by the formula depicted above). The second active agent, in case of potassium chloride, may preferably be comprised in amounts of from 200 to 1000 mg per oral dosage form, such as from 500 to 750 mg, for instance 573 mg. For second active agents other than KCl, other dosage amounts, for instance lower amounts, may also be suitable. Generally, suitable doses of active agents in combination medicaments, such as the present oral dosage form, are often reduced as compared to the doses of the same active agents employed in monotherapy.

According to a further aspect, the present invention provides a method for preparing a tablet for the immediate release of bendroflumethiazide and controlled release of a second active agent, preferably potassium chloride, which is free from polyethylene oxide and polymers comprising polyethylene oxide moieties or more generally polyalkylene oxides and polymers comprising polyalkylene oxide moieties, as described before, comprising the following steps:
a) preparing a tablet core (A) by mixing particles of the second active agent with a first controlled release agent and optionally pharmaceutically acceptable excipient(s) and compressing the mixture to form a tablet core (A), which comprises a controlled release matrix,
   wherein
   a1) the particles of the second active agent may be coated with a second controlled release agent, and step a) optionally further comprises a step of coating the particles of the second active agent with the second controlled release agent or
   a2) wherein the particles of the second active agent are not provided in a form with a coating (are used as such, *i.e.* uncoated)
   or
b) preparing a tablet core (B) by mixing particles of the second active agent with at least one pharmaceutically acceptable excipient and compressing the mixture to form the tablet core (B), and coating the tablet core (B) with a controlled release film (CR1) comprising a first controlled release agent to form a coated tablet core (BCR1),
   wherein
   b1) the particles of the second active agent may be coated with a second controlled release agent, and step b) optionally further comprises a step of coating the particles of the second active agent with the second controlled release agent or
   b2) wherein the particles of the second active agent are not provided in a form with a coating (are used as such, i.e. uncoated) and
c) coating the tablet core prepared in step a) or the coated tablet core prepared in step b) with a coating suspension comprising bendroflumethiazide to provide the tablet.

Step a) may further include a step of coating the tablet core (A) with a controlled release film (CR2) comprising a third controlled release agent to form a coated tablet core (ACR2). First and/or second and/or third controlled release agent may be different.

The method may further include a step of coating the tablet core (A) or coated tablet core (BCR1) with a film (IR1) that does not substantially control release of the second active agent from the tablet core to form a coated tablet core. However, it is preferred that no additional coating step is carried out, such that step c) would be the next step after steps a) or b).

For the coating steps, conventional methods known in the art for film-coating a tablet core may be employed. Likewise, conventional methods known in the art may be used for mixing and compressing. The preparation of the tablet core may include dry or wet granulation or merely involve powder blending.

Preferably, in said method, the coating suspension in step c) further comprises a surface-active agent with an HLB value of 12 or more in an amount of 1 part by weight relative to 0.2 to 10 parts by weight of bendroflumethiazide, the surface-active agent preferably being a sucrose ester, as set out in the context of the oral dosage form to be prepared.

With regard to the controlled release agents, pharmaceutically acceptable excipients and further embodiments, reference is made to the description of the oral dosage form herein before.

The thickness of a coating around particles of the second active agent may range from 3 to 30 µm, for instance, preferably from 5 to 20 µm.

Pharmaceutically acceptable solvents may be used in the preparation of the oral dosage forms, in particular tablets, according to the present invention. They include water, ethanol, isopropanol, acetone and mixtures thereof.

In terms of diminished complexity of manufacturing and avoidance of potential interactions between the individual ingredients, it is generally preferable to keep the number of different ingredients low. Therefore, in exemplary embodiments of the present invention, the term "comprising" generally used herein may be substituted for "consisting of", indicating that the presence of additional ingredients shall be excluded.

It shall be mentioned that those embodiments, wherein the second active agent may be comprised in the oral dosage form, more specifically in the second portion thereof, in the form of particles, which particles are coated with a controlled release agent such that the coated particles release the second active agent in a controlled manner are advantageous as such, irrespective of the presence of polyethylene oxide and polymers comprising polyethylene oxide moieties/polyalkylene oxides and polymers comprising polyalkylene oxide moieties. Therefore, in a further aspect, the present invention encompasses an oral dosage form for the immediate release of bendroflumethiazide and controlled release of a second active agent, comprising: a first portion comprising bendroflumethiazide, wherein the first portion is adapted for immediate release of bendroflumethiazide, and a second portion comprising the second active agent, the second portion being separate from the first portion and adapted for controlled release of the second active agent, characterized in that the second active agent is comprised in the form of particles, which particles are coated with a controlled release agent.

Exemplary embodiments of the present invention will be described in the following with reference to the figures, wherein
- Figure 1: is a schematic diagram of a method of preparing tablets according to the first preferred embodiment of the present invention,
- Figure 2: is a schematic diagram of a method of preparing tablets according to the third preferred embodiment of the present invention,

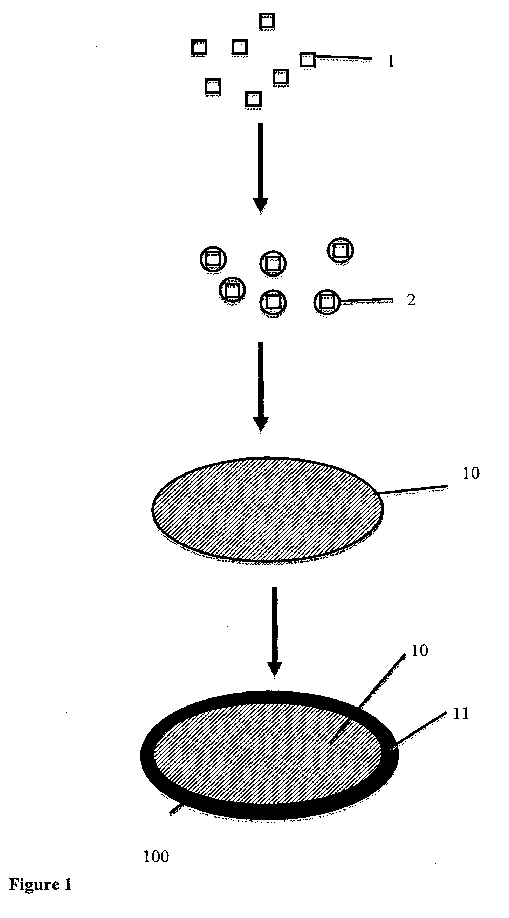

As illustrated in Figure 1, potassium crystals 1 were coated with controlled release agent Eudragit^{®} NM 30 D by spray coating producing coated KCl crystals 2. Apart from Eudragit^{®} NM30D, the coating suspension comprised micronized talc (as a lubricant) and Syloid (as a glidant) in purified water. The exact composition is provided in Table 1 below. The coating process was carried out in fluidized bed (Inlet temperature is: 30-35°C, product temperature: 19-23°C).

After addition of Syloid (from the matrix composition) the coated KCl crystals were cured in fluidized bed by spraying water onto the crystals for 30 min.

The cured coated KCl crystals 2 were then mixed with HPMC as a controlled release agent, pregelatinized starch and magnesium stearate in a V-blender (see Table 1 below). The resulting mixture was then compressed into 1000 mg tablet cores 10 ("A" in the terminology used herein) of the desired dimensions. All steps were controlled by in-process controls and other controls as commonly used in the pharmaceutical industry.

**Table 1: CR tablet core**

| | | **Role** | **Dosage form composition (% w/w)** | |
|---|---|---|---|---|
| | | | **mg/unit** | **%** |
| **Tablet core** | | | | |
| ***KCl coating*** | | | | |
| A | Potassium chloride | Second active agent | 573.00 | 54.15 |
| B | Eudragit^{®} NM30D (dry substance) | Controlled release agent | 85.95 | 8.12 |
| C | Talc micronized | Glidant | 21.49 | 2.03 |
| D | Syloid | Glidant | 21.49 | 2.03 |
| E | Purified water* | Coating solvent | q.s.** | q.s.** |

| ***Matrix*** | | | | |
|---|---|---|---|---|
| F | Syloid | Glidant | 5.00 | 0.47 |
| G | HPMC K100LV | Controlled release agent | 275.00 | 25.99 |
| H | Pregelatinized starch | Filler | 15.57 | 1.47 |
| I | Magnesium stearate | Lubricant | 2.50 | 0.24 |
| **Total tablet core** | | | 1000.0 | 94.5 |

| | | | | |
|---|---|---|---|---|
| * Not present in the final formulation. ** The coating suspension is composed of 15% w/w solids. | | | | |

The tablet cores 10 are then pan coated with a coating suspension comprising bendroflumethiazide and further compounds, as listed in Table 2 below:

**Table 2: IR film coating**

| | | | **Example 1** | **Example 2** |
|---|---|---|---|---|
| | | **Role** | **mg/tablet** | **mg/tablet** |
| A | B endroflumethiazide | Active agent | 2.5 | 1.25 |
| B | Opadry^{®} II White (PEO free variety) | film forming agent | 48.0 | 48.0 |
| C | Talc micronized | Filler | 0.1 | 1.35 |
| D | Ferrous oxide yellow | Colourant | 0.5 | |
| E | Ferrous oxide red | Colourant | 0.5 | |
| F | Sucrose palmitate | Solubility enhancer | 5.0 | 5.0 |
| G | Citric acid monohydrate | PH adjusting agent | 2.5 | 2.5 |
| H | Purified water* | Coating solvent | 562 | 562 |
| Total IR film | | | 58.1 | 58.1 |

| | | | | |
|---|---|---|---|---|
| * Not present in the final formulation. | | | | |

Citric acid and sucrose palmitate were dispersed in about nine tenth of the total amount of purified water and stirred until complete dissolution. Opadry was added and the resulting suspension stirred for 45 minutes. Talc and colourants were dispersed in the remaining amount of water and homogenized for 10 minutes by ultra turrax. The two suspensions were combined, bendroflumethiazide was added and the resulting coating suspension mixed for at least 60 minutes. The resulting coating suspension was then sprayed onto the tablet cores 10 in a perforated drum with an inlet air temperature of 50 to 60°C and subsequently dried until LOD (loss on drying) of the resulting tablets 100 was less than 2.5%. Thus, the resulting tablet 100 comprises a tablet core 10 with a controlled release matrix coated with an immediate release coating 11. All steps were controlled by in-process controls and other controls as commonly used in the pharmaceutical industry.

The tablets according to Examples 1 and 2 compare favourably with the commercially available products of corresponding dosage of bendroflumethiazide (1.25 and 2.5 mg) with respect to storage stability: Stability is improved. Stability was assessed by analysing by HPLC the amount of a characteristic degradation product respectively impurity (Impurity A acc. to the monograph on bendroflumethiazide in Pharm.Eur. 7.0) present in the samples after predetermined time intervals. Storage conditions were: closed bottles with desiccant, 40°C ± 2°C; 75% ± 5% relative humidity. Table 3 below indicates the results obtained:

**Table 3: Stability upon storage**

| | Amount of impurity | | | |
|---|---|---|---|---|
| Sample | initial | 6 weeks | 3 months | 6 months |
| Commercial product 2.5 mg | 0.13% | 0.21% | 0.46% | 0.75% |
| Example 1 (2.5 mg) | 0.055% | n.d. | 0.15% | 0.15% |
| Commercial product 1.25 mg | 0.29% | 0.71% | n.d. | n.d. |
| Example 2 (1.25 mg) | 0.19% | n.d. | 0.26% | 0.23% |

As evident from above Table 3, the examples according to the present invention show improved stability.

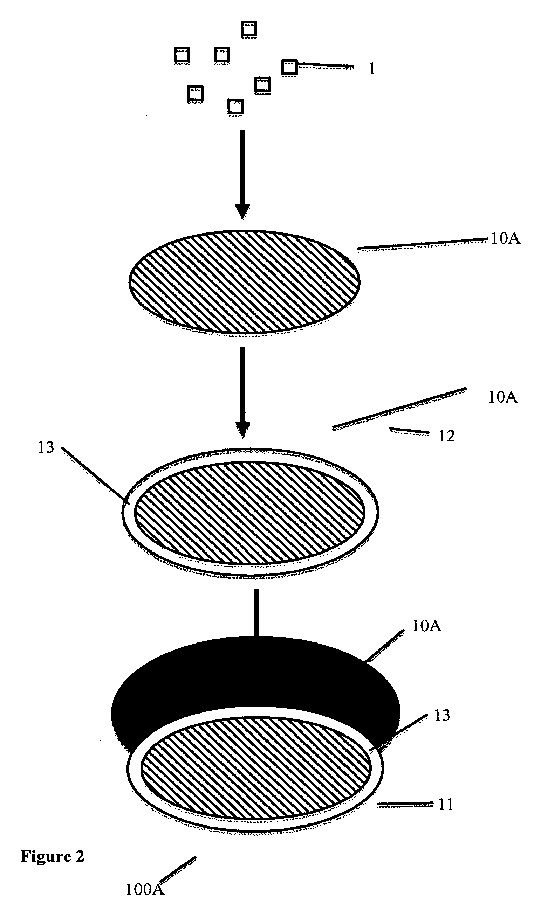

In the alternative embodiment illustrated in Figure 2, KCl crystals 1 are blended with pharmaceutically acceptable excipients and HPMC as controlled release agent and compressed to a tablet core 10A. The tablet core 10A is then coated with a film coating 13 comprising a controlled release agent, such as Eudragit^{®}, which is adapted to release KCl in a controlled manner. The coated tablet core 12 is then coated with an immediate release coating 11 comprising bendroflumethiazide, thus completing the oral dosage form 100A. An exemplary formulation is given in Table 4 below:

**Table 4:**

| | | **Role** | **Dosage form composition** |
|---|---|---|---|
| | | | **mg/unit** |
| **Tablet core** | | | |
| ***Internal phase KCl*** | | | |
| A | Potassium chloride | Second active agent | 573.00 |

| ***External phase*** | | | |
|---|---|---|---|
| B | Syloid | Glidant | 5.00 |
| C | HPMC K100LV | Controlled release agent | 275.00 |
| D | Pregelatinized starch | Filler | 15.57 |
| E | Magnesium stearate | Lubricant | 2.50 |
| **Total tablet core** | | | 871.07 |

| **Controlled release coating** | | | |
|---|---|---|---|
| F | Eudragit^{®} NM30D (dry substance) | Controlled release agent | 10 |
| G | Talc micronized | Glidant | 2.5 |
| H | Syloid | Glidant | 2.5 |
| I | Purified water* | Coating solvent | q.s.** |
| | | | |

| **IR film coating** | | | |
|---|---|---|---|
| as in Example 1 | | | |

| | | | |
|---|---|---|---|
| * Not present in the final formulation. ** The coating suspension is composed of 15% w/w solids | | | |

The present invention therefore encompasses the following aspects and embodiments:
1. Oral dosage form for the immediate release of bendroflumethiazide and controlled release of a second active agent, comprising:
   a first portion comprising bendroflumethiazide, wherein the first portion is adapted for immediate release of bendroflumethiazide,
   and a second portion comprising the second active agent, the second portion being separate from the first portion and adapted for controlled release of the second active agent,
   characterized in that the oral dosage form is free from polyethylene oxide and polymers comprising polyethylene oxide moieties and preferably free from polyalkylene oxides and polymers comprising polyalkylene oxide moieties.
2. Oral dosage form according to item 1, wherein the second active ingredient is potassium chloride.
3. Oral dosage form according to item 1 or 2, wherein the first portion comprises a surface-active agent having an HLB value of 12 or more.
4. Oral dosage form according to item 3, wherein the surface-active agent is a sucrose ester.
5. Oral dosage form according to item 4, wherein the surface-active agent is sucrose palmitate.
6. Oral dosage form according to any preceding item, wherein the oral dosage form is a tablet, and preferably wherein the first portion is a film coating and the second portion is or comprises a tablet core.
7. Oral dosage form according to any preceding item, wherein the second portion comprises particles of the second active agent, which particles are coated with a controlled release agent such that the coated particles are adapted for controlled release of the second active agent.
8. Oral dosage form according to any preceding item, wherein the oral dosage form is a tablet and wherein the second portion is a tablet core comprising the second active agent and at least one pharmaceutically acceptable excipient, and wherein the tablet core is preferably coated with a film comprising a controlled release agent.
9. Oral dosage form according to item 8, wherein the tablet core comprises or consists of a controlled release matrix comprising a controlled release agent.
10. Oral dosage form according to item 9, wherein the controlled release matrix comprises a controlled release agent selected from the group comprising polymers based on at least one of acrylate and methacrylate, cellulose ethers and esters, waxes and lipids, cellulose acetate phthalate (CAP), hydroxypropyl methylcellulose phthalate (HPMCP), hydroxypropyl methylcellulose acetate succinate (HPMC-AS) and polyvinyl acetate phthalate (PVAP).
11. Oral dosage form according to item 7 or 8, wherein the controlled release agent is a polymer based on at least one of acrylate and methacrylate, preferably ethyl acrylate and methyl methacrylate.
12. Oral dosage form according to any preceding item for use in the treatment of hypertension and/or oedema.
13. Method for preparing a tablet for the immediate release of bendroflumethiazide and controlled release of a second active agent according to item 6, preferably potassium chloride, comprising:
   a) preparing a tablet core (A) by mixing particles of the second active agent with a first controlled release agent and optionally pharmaceutically acceptable excipient(s) and compressing the mixture to form a tablet core (A), which comprises a controlled release matrix,
      or
   b) preparing a tablet core (B) by mixing particles of the second active agent with at least one pharmaceutically acceptable excipient and compressing the mixture to form the tablet core (B), and coating the tablet core (B) with a controlled release film (CR1) comprising a first controlled release agent to form a coated tablet core (BCR1),
      and
   c) coating the tablet core prepared in step a) or the coated tablet core prepared in step b) with a coating suspension comprising bendroflumethiazide to provide the tablet.
14. Method according to item 13, wherein the coating suspension further comprises a surface-active agent with an HLB value of 12 or more in an amount of 1 part by weight relative to 0.2 to 5 parts by weight of bendroflumethiazide, preferably 4, 2 or 1 part by weight relative to 1 or 2 parts by weight of bendroflumethiazide, the surface-active agent preferably being a sucrose ester.
15. Method according to item 13, wherein the method further comprises coating the particles of the second active agent with a second controlled release agent prior to preparation of the tablet core (A, B).

## Claims

1. Oral dosage form for the immediate release of bendroflumethiazide and controlled release of a second active agent, comprising:
a first portion comprising bendroflumethiazide, wherein the first portion is adapted for immediate release of bendroflumethiazide,
and a second portion comprising the second active agent, the second portion being separate from the first portion and adapted for controlled release of the second active agent,
**characterized in that** the oral dosage form is free from polyethylene oxide and polymers comprising polyethylene oxide moieties.

2. Oral dosage form according to claim 1, wherein the second active ingredient is potassium chloride.

3. Oral dosage form according to claim 1 or 2, wherein the first portion comprises a surface-active agent having an HLB value of 12 or more.

4. Oral dosage form according to claim 3, wherein the surface-active agent is a sucrose ester.

5. Oral dosage form according to claim 4, wherein the surface-active agent is sucrose palmitate.

6. Oral dosage form according to any of the preceding claims, wherein the oral dosage form is a tablet.

7. Oral dosage form according to any of the preceding claims, wherein the second portion comprises particles of the second active agent, which particles are coated with a controlled release agent such that the coated particles are adapted for controlled release of the second active agent.

8. Oral dosage form according to one of claims 1 to 7, wherein the oral dosage form is a tablet and wherein the second portion is a tablet core comprising the second active agent and at least one pharmaceutically acceptable excipient.

9. Oral dosage form according to claim 8, wherein the tablet core comprises or consists of a controlled release matrix comprising a controlled release agent.

10. Oral dosage form according to claim 9, wherein the controlled release matrix comprises a controlled release agent selected from the group comprising polymers based on at least one of acrylate and methacrylate, cellulose ethers and esters, waxes and lipids, cellulose acetate phthalate (CAP), hydroxypropyl methylcellulose phthalate (HPMCP), hydroxypropyl methylcellulose acetate succinate (HPMC-AS) and polyvinyl acetate phthalate (PVAP).

11. Oral dosage form according to claim 7 or 8, wherein the controlled release agent is a polymer based on at least one of acrylate and methacrylate.

12. Oral dosage form according to any of the preceding claims for use in the treatment of hypertension and/or oedema.

13. Method for preparing a tablet for the immediate release of bendroflumethiazide and controlled release of a second active agentaccording to claim 6, comprising:
a) preparing a tablet core (A) by mixing particles of the second active agent with a first controlled release agent and optionally pharmaceutically acceptable excipient(s) and compressing the mixture to form a tablet core (A), which comprises a controlled release matrix,
or
b) preparing a tablet core (B) by mixing particles of the second active agent with at least one pharmaceutically acceptable excipient and compressing the mixture to form the tablet core (B), and coating the tablet core (B) with a controlled release film (CR1) comprising a first controlled release agent to form a coated tablet core (BCR1),
and
c) coating the tablet core prepared in step a) or the coated tablet core prepared in step b) with a coating suspension comprising bendroflumethiazide to provide the tablet.

14. Method according to claim 13, wherein the coating suspension further comprises a surface-active agent with an HLB value of 12 or more in an amount of 1 part by weight relative to 0.2 to 5 parts by weight of bendroflumethiazide.

15. Method according to claim 13, wherein the method further comprises coating the particles of the second active agent with a second controlled release agent prior to preparation of the tablet core (A, B).

## Patentansprüche

1. Orale Darreichungsform zur sofortigen Freisetzung von Bendroflumethiazid und zur kontrollierten Freisetzung eines zweiten Wirkstoffes, umfassend:
eine erste Portion umfassend Bendroflumethiazid, wobei die erste Portion zur sofortigen Freisetzung von Bendroflumethiazid geeignet ist,
und eine zweite Portion umfassend den zweiten Wirkstoff, wobei die zweite Portion getrennt ist von der ersten Portion und zur kontrollierten Freigabe des zweiten Wirkstoffs geeignet ist,
**dadurch gekennzeichnet, dass** die orale Darreichungsform frei von Polyethylenoxid und Polyethylenoxid-Resten enthaltenden Polymeren ist.

2. Orale Darreichungsform gemäß Anspruch 1, wobei der zweite Wirkstoff Kaliumchlorid ist.

3. Orale Darreichungsform gemäß Anspruch 1 oder 2, wobei die erste Portion einen oberflächenaktiven Stoff mit einem HLB Wert von 12 oder mehr umfasst.

4. Orale Darreichungsform gemäß Anspruch 3, wobei der oberflächenaktive Stoff ein Suchroseester ist.

5. Orale Darreichungsform gemäß Anspruch 4, wobei der oberflächenaktive Stoff Suchrose Palmitat ist.

6. Orale Darreichungsform gemäß einem der vorstehenden Ansprüche, wobei die orale Darreichungsform eine Tablette ist.

7. Orale Darreichungsform gemäß einem der vorstehenden Ansprüche, wobei die zweite Portion Partikel des zweiten Wirkstoffes umfasst, welche mit einem Stoff zur kontrollierten Freisetzung überzogen sind, so dass die überzogenen Partikel zur kontrollierten Freisetzung des zweiten Wirkstoffes geeignet sind.

8. Orale Darreichungsform gemäß einem der Ansprüche 1 bis 7, wobei die orale Darreichungsform eine Tablette ist und wobei die zweite Portion ein Tablettenkern, umfassend den zweiten Wirkstoff und mindestens einen pharmazeutisch annehmbaren Hilfsstoff, ist.

9. Orale Darreichungsform gemäß Anspruch 8, wobei der Tablettenkern eine Matrix zur kontrollierten Freigabe umfassend einen Stoff zur kontrollierten Freigabe umfasst oder daraus besteht.

10. Orale Darreichungsform gemäß Anspruch 9, wobei die Matrix zur kontrollierten Freigabe einen Stoff zur kontrollierten Freigabe umfasst, der ausgewählt ist aus der Gruppe umfassend Polymere, die mindestens auf einem von Acrylat und Methacrylat, Celluloseethern und -estern, Wachsen und Lipiden, Celluloseacetatphthalat (CAP), Hydroxypropylmethylcellulosephthalat (HPMCP), Hydroxypropylmethylcelluloseacetatsuccinat (HPMA-AS) und Polyvinylacetatphthalat (PVAP) basieren.

11. Orale Darreichungsform gemäß Anspruch 7 oder 8, wobei der Stoff zur kontrollierten Freisetzung ein Polymer basierend auf mindestens einem von Acrylat und Methacrylat ist.

12. Orale Darreichungsform gemäß einem der vorstehenden Ansprüche zur Verwendung in der Behandlung von Hypertonie und/oder Ödemen.

13. Verfahren zur Herstellung einer Tablette zur sofortigen Freisetzung von Bendroflumethiazid und zur kontrollierten Freisetzung eines zweiten Wirkstoffes gemäß Anspruch 6, umfassend:
a) Herstellen eines Tablettenkerns (A) durch Mischen der Partikel des zweiten Wirkstoffes mit einem ersten Stoff zur kontrollierten Freisetzung und gegebenenfalls pharmazeutisch annehmbaren Hilfsstoff(en) und Verpressen der Mischung zu einem Tablettenkern (A), welcher eine Matrix zur kontrollierten Freisetzung umfasst,
oder
b) Herstellen eines Tablettenkerns (B) durch Mischen der Partikel des zweiten Wirkstoffes mit mindestens einem pharmazeutisch annehmbaren Hilfsstoff und Verpressen der Mischung zu einem Tablettenkern (B), und Überziehen des Tablettenkerns (B) mit einem Film zur kontrollierten Freisetzung (CR1), umfassend einen ersten Stoff zur kontrollierten Freisetzung, um einen überzogenen Tablettenkern (BCR1) zu erhalten,
und
c) Überziehen des in Schritt a) hergestellten Tablettenkerns oder des in Schritt b) hergestellten überzogenen Tablettenkerns mit einer Überzugslösung umfassend Bendroflumethiazid, um die Tablette zu erhalten.

14. Verfahren gemäß Anspruch 13, wobei die Überzugslösung ferner einen oberflächenaktiven Stoff mit einem HLB Wert von 12 oder mehr in einer Menge von 1 Gewichtsteil gegenüber 0,2 bis 5 Gewichtsteilen Bendroflumethiazid umfasst.

15. Verfahren gemäß Anspruch 13, wobei das Verfahren weiterhin das Überziehen der Partikel des zweiten Wirkstoffes mit einem zweiten Stoff zur kontrollierten Freisetzung vor der Herstellung des Tablettenkerns (A, B) umfasst.

## Revendications

1. Forme posologique orale pour la libération immédiate de bendrofluméthiazide et la libération contrôlée d'un second agent actif, comprenant:
une première partie comprenant du bendrofluméthiazide, dans laquelle la première partie est adaptée à la libération immédiate de bendrofluméthiazide,
et une seconde partie comprenant le second agent actif, la seconde partie étant séparée de la première partie et adaptée pour la libération contrôlée du second agent actif,
**caractérisé en ce que** la forme posologique orale est exempte d'oxyde de polyéthylène et de polymères comprenant des motifs d'oxyde de polyéthylène.

2. Forme posologique orale selon la revendication 1, dans laquelle le second ingrédient actif est le chlorure de potassium.

3. Forme posologique orale selon la revendication 1 ou 2, dans laquelle la première partie comprend un agent tensioactif ayant une valeur HLB de 12 ou plus.

4. Forme posologique orale selon la revendication 3, dans laquelle l'agent tensioactif est un ester de saccharose.

5. Forme posologique orale selon la revendication 4, dans laquelle l'agent tensioactif est le palmitate de saccharose.

6. Forme posologique orale selon l'une quelconque des revendications précédentes, dans laquelle la forme posologique orale est un comprimé.

7. Forme posologique orale selon l'une quelconque des revendications précédentes, dans laquelle la deuxième partie comprend des particules du second agent actif, les particules étant revêtues avec un agent de libération contrôlée de telle sorte que les particules revêtues sont adaptés pour une libération contrôlée du second agent actif.

8. Forme posologique orale selon l'une des revendications 1 à 7, dans laquelle la forme posologique orale est un comprimé et dans lequel la deuxième partie est un noyau de comprimé comprenant le second agent actif et au moins un excipient pharmaceutiquement acceptable.

9. Forme posologique orale selon la revendication 8, dans laquelle le noyau de comprimé comprend ou consiste en une matrice à libération contrôlée comprenant un agent de libération contrôlée.

10. Forme posologique orale selon la revendication 9, dans laquelle la matrice à libération contrôlée comprend un agent de libération contrôlée choisie parmi le groupe comprenant des polymères à base d'au moins un d'acrylate et de méthacrylate, d'éthers et esters de cellulose, de cires et lipides, d'acétophtalate de cellulose (CAP), de phtalate d'hydroxypropylméthylcellulose (HPMCP), d'acétosuccinate d'hydroxypropylméthylcellulose (HPMC-AS) et d'acetate phtalate de polyvinyle (PVAP).

11. Forme posologique orale selon la revendication 7 ou 8, dans laquelle l'agent de libération contrôlée est un polymère à base d'au moins un d'acrylate et de méthacrylate.

12. Forme posologique orale selon l'une quelconque des revendications précédentes pour usage dans le traitement de l'hypertension et / ou de l'oedème.

13. Procédé de préparation d'un comprimé pour la libération immédiate de bendrofluméthiazide et la libération contrôlée d'un second agent actif selon la revendication 6, comprenant:
a) la préparation d'un noyau de comprimé (A) en mélangeant des particules du second agent actif avec un premier agent de libération contrôlée et facultativement avec un ou plusieurs excipient(s) pharmaceutiquement acceptable(s) et la compression du mélange afin de former un noyau de comprimé (A), qui comprend une matrice à libération contrôlée,
ou
b) la préparation d'un noyau de comprimé (B) en mélangeant des particules du second agent actif avec au moins un excipient pharmaceutiquement acceptable et la compression du mélange afin de former le noyau de comprimé (B), et le revêtement du noyau de comprimé (B) avec un film à libération contrôlée (CR1) comprenant un premier agent de libération contrôlée afin de former un noyau de comprimé revêtu (BCR1),
et
c) le revêtement du noyau du comprimé préparé dans l'étape a) ou du noyau du comprimé revêtu préparé à l'étape b) avec une suspension de revêtement comprenant du bendrofluméthiazide afin de fournir le comprimé.

14. Procédé selon la revendication 13, dans lequel la suspension de revêtement comprend en outre un agent tensioactif ayant une valeur HLB de 12 ou plus en une quantité de 1 partie en poids par rapport à 0,2 à 5 parties en poids de bendrofluméthiazide.

15. Procédé selon la revendication 13, dans lequel le procédé comprend en outre le revêtement des particules du second agent actif avec un second agent de libération contrôlée avant la préparation du noyau de comprimé (A, B).
